# EUROPÄISCHE PATENTSCHRIFT

(11) **EP 3 526 315 B1**
(45) Veröffentlichungstag und Bekanntmachung des Hinweises auf die Patenterteilung: **25.05.2022**
(21) Anmeldenummer: 17803790.9
(22) Anmeldetag: 13.10.2017
(51) Int. Cl.: C10L 3/08, C07C 1/00, C07C 1/04, C25B 1/00, C25B 1/02, C25B 15/08

(54) **VERFAHREN ZUR HERSTELLUNG VON METHAN**
METHOD FOR PRODUCING METHANE
PROCÉDÉ POUR LA PRODUCTION DE MÉTHANE

(30) Priorität: 13.10.2016 DE 102016219986
(43) Veröffentlichungstag der Anmeldung: 21.08.2019
(73) Patentinhaber: FLD Technologies GmbH, 63071 Offenbach am Main (DE)
(72) Erfinder: FULDE, Marek, 63071 Offenbach (DE)
(74) Vertreter: Hörschler, Wolfram Johannes
(86) Internationale Anmeldenummer: PCT/EP2017/076200
(87) Internationale Veröffentlichungsnummer: WO 2018/069503

(56) Entgegenhaltungen:
- WO-A1-2008/097691
- WO-A2-2007/005284
- CN-U- 201 962 264
- DE-A1-102012 200 221
- DE-A1-102014 001 933
- YUN ZHENG ET AL: "A review of high temperature co-electrolysis of H 2 O and CO 2 to produce sustainable fuels using solid oxide electrolysis cells (SOECs): advanced materials and technology", CHEMICAL SOCIETY REVIEWS, Bd. 46, Nr. 5, 1. Januar 2017 (2017-01-01) , Seiten 1427-1463, XP055448645, ISSN: 0306-0012, DOI: 10.1039/C6CS00403B

## Beschreibung

Die Erfindung betrifft ein Verfahren zur Herstellung von Methan, wobei ein erster Massenstrom, der Wasserstoff enthält, mit einem zweiten Massenstrom, der Kohlendioxid und/oder Kohlenmonoxid enthält, vermischt wird und Wasserstoff sowie Kohlendioxid und/oder Kohlenmonoxid im erhaltenen Gemisch in einer katalytischen Methanisierungsreaktion in Methan umgesetzt wird.

Mit "ower-to-Gas" (PtG) wird ein Verfahren bezeichnet, in dem mit Hilfe des überschüssigen, aus erneuerbaren Quellen stammenden, elektrischen Stroms Methangas erzeugt wird. Methangas gilt als Speichermedium für die erneuerbare Energie. Im ersten Schritt wird die Wasserelektrolyse unter Bildung von Wasserstoff und Sauerstoff durchgeführt. Wegen fehlender Infrastruktur kann der entstehende Wasserstoff nur begrenzt gespeichert werden. Der Speichereffekt wird dadurch erreicht, dass Wasserstoff unter Einwirkung von Kohlendioxid in Methangas umgewandelt wird. Kohlendioxid stammt aus der Verbrennung fossiler Brennstoffe, aus der Biogas-Produktion, aus anderen industriellen Prozessen (z.B. Zement-, bzw. Stahlerzeugung) oder aus der Adsorption aus der Luft. Das auf diesem Wege erzeugte Methangas wird als synthetisches Erdgas in das öffentliche Gasnetz eingespeist und für energetische Zwecke (z. B. Verbrennung) benutzt. Durch Verwendung des Erdgas-Netzes ist es möglich, Methangas zu transportieren und zu speichern. Die Effektivität der Speicherung wird dabei als Verhältnis des Wasserstoffs definiert, der von außen dem System zugefügt wird und nach Durchlaufen des Speicherungsprozesses gebunden in Form von Methan zur Verfügung steht.

Die dem PtG-Verfahren zugrundeliegende chemische Reaktion, die Sabatier-Reaktion, liefert neben Methan die zweifache Mol-Menge Wasser:

CO₂ + 4 H₂ -> CH₄ + 2 H₂O (1)

Als Wasserstoffträger wird ausschließlich Methan eingesetzt. Wasser wird abgetrennt und der Elektrolyse wieder zugeführt. Wie der Reaktionsgleichung zu entnehmen ist, wird gemäß dem Vorgehen nur die Hälfte des in der Elektrolyse gewonnen Wasserstoffs in das speicherbare Methan umgewandelt. Zwei von vier für die Methanisierung benötigten H₂-Molekülen gehen als Wasser "verloren".

DE102012021256A1 beschreibt einen zweistufigen Prozess zur Speicherung von erneuerbaren Energien. Im ersten Schritt wird Kohlenstoff unter Einwirkung von Wasser in Synthesegas umgewandelt. Sofern der Prozess autotherm, das heißt ohne zusätzliche Wärmezufuhr geführt wird, entsteht folgendes Gasgemisch:

3C + H₂O + O₂ -> 3 CO + H₂ (2)

Im zweiten Schritt wird die Methanisierung des Gasgemisches mit dem zu speichernden Wasserstoff (H₂) aus der Wasser-Elektrolyse vorgenommen:

(3 CO + H₂) + 8 H₂ -> 3 CH₄ + 3 H₂O (3)

Zusammenfassend werden 12 von 16 Wasserstoff-Atomen zur Methan-Bildung verwendet, das heißt 75% des zu speichernden, bei der Wasser-Elektrolyse gewonnenen Wasserstoffs werden gespeichert.

In DE 34 244 24 A1 ist ein Verfahren beschrieben, bei dem zuerst Wasserstoff aus Koksofengas abgetrennt wird, so dass ein für eine nachfolgende Methanisierung geeignetes Verhältnis von H₂ zu CO eingestellt wird. In einem zweiten Prozessschritt wird mittels eines Katalysators die Herstellung von Methangas vorgenommen. Methangas wird als synthetisches Erdgas in das öffentliche Gasnetz eingespeist, verbrannt und für energetische Zwecke genutzt.

In DE 35 152 50 A1 sowie DE 38 05 397 A1 sind Verfahren beschrieben, in denen das H₂-reiche Koksofengas mit CO-haltigem Hüttengas vermischt wird, um das stöchiometrische, für die Herstellung von Methanol notwendige Verhältnis einzustellen. Man setzt hierbei voraus, dass Hüttengas in ausreichenden Mengen zur Verfügung steht.

DE 10 2012 200 221 A1 offenbart ein Verfahren zur Erzeugung eines methanreichen Gases mit den Schritten: (a) Erzeugen von Synthesegas aus Biomasse; und (b) katalytisches Umwandeln des Synthesegases unter Anreicherung mit Wasserstoff, wodurch ein feuchtes methanreiches Gas erzeugt wird.

CN 201962264 U beschreibt ein Verfahren zur Herstellung von Erdgas durch Integration einer durch Windkraft betriebenen Wasserelektrolyse mit einer herkömmlichen Kohlevergasung.

Die DE 10 2014 001 933 A1 betrifft ein Verfahren und eine Anlage zum Erzeugen von Biomethan, mit einer Methanisierungseinheit, in der Kohlenstoffdioxid, Kohlenmonoxid und Wasserstoff in Methan und Wasser umgewandelt werden. Ferner ist eine Elektrolyseeinrichtung vorgesehen, in der der Elektrolyseeinrichtung zugeführtes Wasser durch elektrischen Strom in Wasserstoff und Sauerstoff gespalten wird, wobei der mittels der Elektrolyseeinrichtung erzeugte Wasserstoff wenigstens zum Teil der Methanisierungseinheit zugeführt wird. Zudem ist eine Biogasanlage vorgesehen, in der durch Vergärung von vergärbaren Stoffen in wenigstens einem Fermenter der Biogasanlage ein wenigstens Methan und Kohlendioxid enthaltendes Biogas erzeugt wird, das wenigstens zum Teil der Methanisierungseinheit zugeführt wird. Weiter ist eine Vergasungseinrichtung vorgesehen, in der durch Vergasung von vergasbaren Stoffen mittels eines Vergasungs- und/oder Oxidationsmittels in wenigstens einem Reaktor der Vergasungseinrichtung ein wenigstens Kohlenmonoxid, Wasserstoff, Kohlendioxid und Methan enthaltendes Synthesegas erzeugt wird, das wenigstens zum Teil der Methanisierungseinheit zugeführt wird, wobei das in der Methanisierungseinheit gebildete Biomethan einer Verwertungs- und/oder Speichereinrichtung, insbesondere einem Gasspeicher und/oder einem Gasnetz, zugeführt wird.

Die WO 2008/097691 A beschreibt eine Vorrichtung und ein Verfahren zur Herstellung von Erdgassubstitut und Elektrizität unter Vermeidung der Produktion von Treibhausgasen, umfassend eine Dampf-Wasserstoffvergasung zur Herstellung eines erdgashaltigen Gasstroms und eines Verkohlungsrückstands, und einen Sauerstoffbrenner zur Verbrennung der Verkohlungsrückstände zum Zwecke der Bildung von Kohlenoxiden. Die Vorrichtung umfasst darüber hinaus eine Algenfarm zur Umsetzung der Kohlenoxide zu Kohlenwasserstoffmaterialien und Sauerstoff.

Die Wasserstoff-Speicherung ist ein wesentlicher Teil des Power-to-Gas-Verfahrens, das zur langfristigen Speicherung erneuerbarer Energien angewandt wird. Aufgabe der Erfindung ist es, ein Verfahren bereitzustellen, welches eine effizientere Nutzung von als Abfall in industriellen Herstellprozessen anfallendem Wasserstoff und an Wasserstoff reichen Gasen erlaubt.

### Offenbarung der Erfindung

Es wird ein Verfahren zur Herstellung von Methan vorgeschlagen, bei dem ein erster Massenstrom G1, der ein Wasserstoff enthaltenes Gasgemisch ist, mit einem zweiten Massenstrom G2, der Kohlendioxid und/oder Kohlenmonoxid enthält, vermischt wird und Wasserstoff sowie Kohlendioxid und/oder Kohlenmonoxid im erhaltenen Gemisch in einer katalytischen Methanisierungsreaktion in Methan umgesetzt wird. Der zweite Massenstrom G2 wird erhalten durch:
a) Durchführen einer katalytischen Hydromethanisierung, bei der Kohlenstoff, Wasserdampf und Sauerstoff als Edukte zu Methan, Kohlenmonoxid und Kohlendioxid bei einer Temperatur im Bereich von 400 °C bis 800 °C in Gegenwart mindestens eines Katalysators umgesetzt werden, oder
b) Durchführen einer Dampf-Wasserstoffvergasung, bei der Kohlenstoff, Wasserstoff, Wasserdampf und Sauerstoff als Edukte zu Methan, Kohlenmonoxid und Kohlendioxid bei einer Temperatur im Bereich von 800 °C bis 1100 °C umgesetzt werden.

Dabei wird bei der katalytischen Hydromethanisierung der Alternative a) oder bei der Dampf-Wasserstoffvergasung gemäß Alternative b) Wasserstoff als zusätzliches Edukt zugegeben, um das Reaktionsgleichgewicht zugunsten des Methans einzustellen, wobei der Wasserstoff aus dem ersten Massenstrom G1 abgetrennt wird.

Der mit dem Verfahren zu speichernde Wasserstoff wird über den ersten Massenstrom G1 bereitgestellt. Der erste Massenstrom G1 wird dem Verfahren als Edukt bzw. Edukt-Gemisch zur Verfügung gestellt.

Bei dem ersten Massenstrom G1 kann es sich um ein Reaktionsprodukt handeln, welches als Abfall in industriellen Prozessen entsteht. Beispielsweise fallen bei der Chlor-Alkali-Elektrolyse oder bei der Koks-Herstellung wasserstoffreiche Gase bzw. Gasgemische an, welche mit dem erfindungsgemäßen Verfahren verwertet werden können. Kokerei-Gas (KG) beispielsweise ist reich an Wasserstoff und enthält zusätzlich Methan, Stickstoff, Kohlenoxide und gegebenenfalls weitere Kohlenwasserstoffe.

Gegebenenfalls werden Gasgemische zunächst aufbereitet, bevor diese als erster Massenstrom G1 eingesetzt werden. Bei Einsatz von Kokerei-Gas werden Rahmen einer üblichen Aufbereitung Entteerung, Entschwefelung, Ammoniakwäsche und/oder Entbenzolung eingesetzt.

Bei dem vorgeschlagenen Verfahren wird die Ausbeute an Methan in Bezug auf den mit dem ersten Massenstrom G1 eingegebenen Wasserstoff dadurch verbessert, dass bereits bei der Erzeugung des zweiten Massenstroms G2 eine Reaktion eingesetzt wird, durch die Methan entsteht und/oder dass bei der Erzeugung des zweiten Massenstroms G2 eine Reaktion eingesetzt wird, die den zur nachfolgenden Methanisierung des im ersten Massenstrom G1 enthaltenen Wasserstoffs benötigten Kohlenstoff in Form von Kohlenmonoxid bereitstellt.

Die Methanisierung gemäß Reaktionsgleichung (1) lässt sich in zwei parallel laufende Reaktionen aufteilen:

H₂ + CO₂ -> CO + H₂O (1a)

3H₂ + CO-> CH₄+H₂O (1b)

Wird der Kohlenstoff nicht in Form von Kohlendioxid (CO₂) sondern in Form von Kohlenmonoxid (CO) bereitgestellt, so entfällt die Reaktion (1a), bei der ein Molekül H₂ in Wasserdampf umgesetzt wird und somit nicht mehr für die Speicherung in Form von Methan zur Verfügung steht. Durch den Einsatz von Kohlenmonoxid als Kohlenstoffquelle wird somit die Speichereffizienz erhöht.

Alternativ oder zusätzlich zur Bereitstellung des Kohlenstoffs in Form von Kohlendioxid werden bei der Bereitstellung des Massenstroms G2 Prozesse eingesetzt, bei denen Kohlenstoff als Edukt nicht nur in Kohlendioxid und/oder Kohlenmonoxid, sondern auch zumindest teilweise in Methan umgesetzt wird. Hierdurch wird der Verlust an Wasserstoff in Form von Wasserdampf bei der nachfolgenden katalytischen Methanisierungsreaktion vollständig oder zumindest teilweise kompensiert.

Nach der Erzeugung des Massenstroms G2 werden die beiden Massenströme G1 und G2 miteinander vermischt. Dabei wird das Mischungsverhältnis der beiden Massenströme G1 und G2 zueinander so eingestellt, dass sich ein für die anschließende katalytische Methanisierungsreaktion optimales stöchiometrisches Verhältnis von Kohlendioxid zu Wasserstoff beziehungsweise Kohlenmonoxid zu Wasserstoff einstellt. Optimal ist ein Verhältnis von vier Wasserstoffmolekülen auf ein Kohlendioxidmolekül bzw. von drei Wasserstoffmolekülen auf ein Molekül Kohlenmonoxid.

Bei der Alternative a) des Verfahrens wird der zweite Massenstrom G2 mittels einer katalytischen Hydromethanisierung erzeugt. Bei der katalytischen Hydromethanisierung werden als Edukte Kohlenstoff, Wasserdampf und eine geringe Menge Sauerstoff bei einem Druck im Bereich von 3 MPa bis 7 MPa und einer Temperatur im Bereich von 400 °C bis 800 °C in Gegenwart eines Katalysators umgesetzt, wobei Methan und Kohlendioxid entstehen. Bevorzugt beträgt der Druck im Bereich von 5,5 MPa bis 6,5 MPa. Die Temperatur liegt bevorzugt im Bereich von 500°C bis 600°C. Als Katalysator wird bevorzugt ein alkalischer Katalysator bestehend aus einer Mischung von mindestens zwei Substanzen aus der Gruppe Na₂CO₃, K₂CO₃, Rb₂CO₃, Li₂CO₃, Cs₂CO₃, KNO₃, K₂SO₄, NaOH, KOH oder Mineralien, die diese Substanzen enthalten, verwendet. Zusätzlich werden der Katalysatormischung Kalziumoxid oder Kalziumhydroxid beigemischt, an die das in der Reaktion entstehende Kohlendioxid zeitweise gebunden wird.

Dies erlaubt die Synthese von Methan und eliminiert weitestgehend Kohlenmonoxid aus dem Produktgemisch. Geeignete Katalysatormaterialien sind beispielsweise aus WO 2007/005284 A2 bekannt.

Die ablaufenden Reaktionen können durch folgende Reaktionsgleichungen beschrieben werden, wobei deren Gleichgewichte von den Reaktionsbedingungen, insbesondere vom Druck und der Temperatur aber auch von Sauerstoffanteil abhängig sind:

6 C + 6 H₂O -> 3 CH₄ + 3 CO₂ (4)

x C + × O₂ -> × CO₂ (5)

Der Sauerstoffanteil x der Reaktion (5) liegt üblicherweise im Bereich von 0,1 Mol bis 0,75 Mol bezogen auf 6 Mol Kohlenstoff der Reaktion (4). Um das Gleichgewicht der Reaktion (4) zugunsten des Methans einzustellen, wird bei der Herstellung des zweiten Massenstroms G2 zusätzlich Wasserstoff zu den Edukten zugegeben. Der Wasserstoff wird dazu dem ersten Massenstrom G1, bei dem es sich um ein Wasserstoff enthaltendes Gasgemisch handelt, entnommen. Hierzu wird ein Teil des Wassersoffs aus dem Gemisch abgetrennt und der Herstellung des zweiten Massenstroms G2 zugeführt. Nach der Herstellung des zweiten Massenstroms G2 wird der verbleibende Teil des ersten Massenstroms G1 mit dem zweiten Massenstrom G2 vermischt.

Nach dem Herstellen des zweiten Massenstroms G2 wird dieser mit dem ersten Massenstrom G1 vermischt, wobei bevorzugt ein optimales stöchiometrisches Verhältnis zwischen CO₂ und H₂ bzw. CO₂ und H₂ eingestellt wird. Nach dem Vermischen des erzeugten zweiten Massenstroms G2 mit dem ersten Massenstrom G1 wird die katalytische Methanisierung von CO₂ mit Wasserstoff vorgenommen.

Die katalytische Methanisierung wird bevorzugt bei Temperaturen im Bereich von 250°C bis 650°C und Druck im Bereich von 0,1 MPa bis 8 MPa durchgeführt. Besonders bevorzugt wird eine Temperatur im Bereich von 250 bis 300 °C gewählt.

Der Druck wird besonders bevorzugt im Bereich von 2,5 MPa bis 3,5 MPa gewählt und beträgt beispielsweise 3 MPa. Als Katalysator können alle Metalle der achten Nebengruppe des Periodensystems Anwendung finden, bevorzugt wird Nickel eingesetzt. Als Trägermaterial werden bevorzugt aktivierte Aluminium-/Magnesium-Oxide verwendet. Die Durchführung der katalytischen Methanisierung ist dem Fachmann bekannt und ist beispielsweise in S. Rönsch, A. Ortwin, "Methanisierung von Synthesegasen - Grundlagen und Verfahrensentwicklung", Chemie-Ingenieur-Technik, 2011, 86, No. 8, 1201 oder in T. Schaaf, J. Grünig, M. Schuster, A. Orth, "Speicherung von elektrischer Energie im Erdgasnetz - Methanisierung von CO2-haltigen Gasen", Chemie-Ingenieur-Technik, 2014, 86, No. 4, 478 beschrieben.

Im Fall von reinem Wasserstoffgas als ersten Massenstrom G1 (nicht erfindungsgemäß) und
Sauerstoffanteil x=0,1 gilt:

3,1CO₂ + 12,4 H₂ -> 3,1 CH₄ + 6,2 H₂O (6)

Zusammenfassend werden auf diese Weise mit Bezug auf die im ersten Massenstrom G1 enthaltene Menge an Wasserstoff ca. 98-99 % des Wasserstoffs in Form von Methan gespeichert.

Wird als erster Massenstrom G1 ein an Wasserstoff reiches Gemisch wie beispielsweise Kokereigas als Wasserstoffquelle eingesetzt, erfolgt neben der Speicherung eine Wiedergewinnung des Wasserstoffs als Wertstoff aus dem Abfall und Überführung in das als Speichermedium genutzte Methan.

Das Kokereigas liegt je nach Herstellverfahren und Kohlesorte in variierender Zusammensetzung vor, wobei der Anteil an Methan von 24 Mol-% bis 35 Mol-%, der Anteil an Kohlenmonoxid von 4 Mol-% bis 7 Mol-%, der Anteil an Kohlendioxid von 2 Mol-% bis 3 Mol-%, der Anteil an Wasserstoff von 50 Mol-% bis 55 Mol-% und der Anteil an Stickstoff von 5 Mol-% bis 12 Mol-% betragen kann. Weitere Stoffe können im Kokereigas möglicherweise vorkommen, beispielsweise Sauerstoff im Bereich von 0,2 bis 0,4 Mol-%, Ammoniak mit einem Anteil von <0,02 Mol-% oder Schwefelwasserstoff mit einem Anteil von < 0,09 Mol-%. Die Summe der Prozentangaben addiert sich zu 100 Mol-%. Ein Beispiel für ein geeignetes Kokerei-Gas ist ein Gemisch gemäß Tabelle 1.

**Tabelle 1**

| | |
|---|---|
| CH₄ | 28 Mol-% |
| CO | 5 Mol-% |
| CO₂ | 2 Mol-% |
| H₂ | 55 Mol-% |
| N₂ | 10 Mol-% |

Zur Überführung des im Kokereigas enthaltenen Kohlendioxids (2 Mol) und Kohlenmonoxid (5 Mol) in Methangas werden 23 Mol Wasserstoff verbraucht. Um die restlichen 32 Mol KG-Wasserstoff zu binden, werden gemäß der Gleichungen (4) und (5) mit einem Sauerstoffanteil von 0,1 Mol 8,01 Mol Kohlendioxid und 7,75 Mol Methan aus der Hydromethanisierung (zweiter Massenstrom G2) hinzugefügt. In die sich anschließenden katalytische Methanisierung wird somit nach Mischung der beiden Massenströme G1 und G2 ein Edukt-Gemisch gemäß Tabelle 2 eingegeben.

**Tabelle 2**

| | |
|---|---|
| CH₄ | 35,84 Mol (28 Mol aus KG (Massenstrom G1) und 7,75 Mol aus Massenstrom G2) |
| H₂ | 55 Mol aus KG (Massenstrom G1) |
| CO | 5 Mol aus KG (Massenstrom G1) |
| CO₂ | 10 Mol (2 Mol aus KG (Massenstrom G1) und 8 Mol aus Massenstrom G2) |
| N₂ | 10 Mol aus KG (Massenstrom G1) |

Die in der Kohle möglicherweise vorhandene Asche, die für die beschriebenen Gasreaktionen inert ist und in die Gasphase nicht übergeht, wurde in den Angaben zur Zusammensetzung der Gasphase weggelassen.

Nach der Umwandlung des als Beispiel angegebenen Gemischs in Tabelle 2 wird ein Produktgemisch aus 50,75 Mol CH₄ und 10 Mol N₂ erhalten, was einer H₂ Speichereffektivität von 92% entspricht.

Bei der Alternative b) des Verfahrens wird eine Dampf-Wasserstoffvergasung zur Erzeugung des zweiten Massenstroms G2 eingesetzt.

Die Dampf-Wasserstoffvergasung wird in zwei Etappen durchgeführt. In einer ersten Etappe reagiert Kohlenstoff mit Wasserdampf und Sauerstoff bei einem Druck im Bereich von 3 MPa bis 7 MPa, und einer Temperatur im Bereich von 800 °C bis 1100°C. Hierbei wird ein Teil des Kohlenstoffs verbrannt und gleichzeitig Wasser in Wasserstoff gespalten. Bevorzugt liegt der Druck im Bereich von 4,5 MPa bis 5,5 MPa. Die Temperatur liegt bevorzugt im Bereich von 900°C bis 1000°C. Es erfolgt anschließend in einer zweiten Etappe die Hydrierung des Kohlenstoffs zu Methan. Bei der zweiten Etappe wird zum Verschieben des Reaktionsgleichgewichts zugunsten von Methan Wasserstoff als weiteres Edukt zugeführt. Beide Etappen können in einem Apparat ausgeführt werden, wobei zuerst Wasserdampf und Sauerstoff und erst im Anschluss zeitlich versetzt Wasserstoff zugegeben wird. Das Verfahren kann durch folgende Reaktionen vollständig beschreiben werden, wobei deren Gleichgewichte von den Reaktionsbedingungen wie beispielsweise Druck und Temperatur abhängig sind:

C + O₂ -> CO₂ (7)

C + H₂O -> CO + H₂ (8)

CO + H₂O -> CO₂ + H₂ (9)

C + 2 H₂ -> CH₄ (10)

In der zweiten Etappe wird dem Reaktionssystem als zusätzliches Edukt Wasserstoff zugefügt, um zusätzlich zur Wahl von Temperatur und Druck das Reaktionsgleichgewicht zugunsten der Bildung von Methan zu verschieben. Durch die Zugabe von Wasserstoff der zweiten Etappe steigt der Wasserstoff-Partialdruck, was die Produktion von Methan begünstigt. Der Wasserstoff wird dazu dem ersten Massenstrom G1, bei dem es sich um ein Wasserstoff enthaltendes Gasgemisch handelt, entnommen. Hierzu wird ein Teil des Wassersoffs aus dem Gemisch abgetrennt und der Herstellung des zweiten Massenstroms G2 zugeführt. Nach der Herstellung des zweiten Massenstroms G2 wird der verbleibende Teil des ersten Massenstroms G1 mit dem zweiten Massenstrom G2 vermischt.

Als Ergebnis der Reaktion entsteht ein Gemisch, welches CH₄, CO, CO₂ und Wasser H₂O enthält. Insbesondere wenn zusätzlich auch Wasserstoff als Edukt zugegeben wurde, verbleibt auch ein Teil des nicht bei der Rektion verbrauchten Wasserstoffs im entstandenen zweiten Massenstrom G2.

Die Zugabe vom Wasserstoff bei der Herstellung des zweiten Massenstroms G2 ist besonders bevorzugt, um das Gleichgewicht der Reaktionen zur Methan-Herstellung hin zu verschieben. Auf diese Weise lässt sich nicht nur der durch Wasserspaltung gewonnene Wasserstoff, sondern auch bereits ca. 30% bis 35% des zugegebenen Wasserstoffs in Methan umwandeln.

Die genaue Zusammensetzung des erhaltenen zweiten Massenstroms G2 ist von den Reaktionsbedingungen abhängig. Eine typische Zusammensetzung eines mit der Dampf-Wasserstoffvergasung erhaltenen zweiten Massenstroms G2 ist in Tabelle 3 angegeben. Zusätzlich können Kleinstmengen (kleiner als 0,3 Mol-%) von beispielsweise Ammoniak, Schwefelwasserstoff, die im Kohlenstoff vorhanden sind, vorkommen. Die Summe der Anteile addiert sich zu 100 Mol-%.

Die in der Kohle vorhandene Asche, die für die beschriebenen Gasreaktionen inert ist und in die Gasphase nicht übergeht, wurde in den Angaben zur Zusammensetzung der Gasphase weggelassen.

**Tabelle 3**

| | |
|---|---|
| H₂ | 42 Mol-% bis 52 Mol-%, |
| H₂O | 14 Mol-% bis 23 Mol-%, |
| CO | 11 Mol-% bis 15 Mol-%, |
| CO₂ | 2 Mol-% bis 6 Mol-%, |
| CH₄ | 14 Mol-% bis 21 Mol-%, |

Die Erfindung wird durch das Beispiel und die Ansprüche erläutert.

### Beispiel:

Bei einer Temperatur von ca. 950°C und einem Druck von 5 MPa wird die Dampf-Wasserstoffvergasung zur Herstellung des zweiten Massenstroms G2 durchgeführt, wobei als Produkt ein Gasgemisch mit der Zusammensetzung H₂ 47 Mol-%, H₂O 18 Mol-%, CO 14 Mol-%, CO₂ 4 Mol-% und CH₄ 18 Mol-% erhalten wird. In der Tabelle 4a sind die Mengen der dabei eingesetzten Edukte Kohlenstoff, Sauerstoff, Wasserstoff und Wasserdampf in mol angegeben. In der Tabelle 4b sind die Verhältnisse der Produkte zueinander in Mol-% angegeben. In den Tabellen 4a und 4b sind jeweils auch die Mengen bzw. die Verhältnisse der entstehenden Produkte angegeben.

**Tabelle 4a**

| | Edukte in Mol | Produkte in Mol |
|---|---|---|
| C (Kohlenstoff) | 1,82 | - |
| O₂ | 0,15 | - |
| H₂ | 2,41 | 1,60 |
| H₂O | 1,00 | 0,60 |
| CO | - | 0,46 |
| CO₂ | - | 0,12 |
| CH₄ | - | 0,605 |

**Tabelle 4b**

| | Edukte in Mol-% | Produkte in Mol-% |
|---|---|---|
| C (Kohlenstoff) | 34 | - |
| O₂ | 3 | - |
| H₂ | 45 | 47 |
| H₂O | 19 | 18 |
| CO | - | 14 |
| CO₂ | - | 4 |
| CH₄ | - | 18 |

Nach dem Herstellen des zweiten Massenstroms G2 wird dieser mit dem ersten Massenstrom G1 vermischt, wobei bevorzugt ein optimales stöchiometrisches Verhältnis zwischen CO₂ und H₂ bzw. CO und H₂ eingestellt wird.

Nach dem Vermischen des erzeugten zweiten Massenstroms G2 mit dem ersten Massenstrom G1 wird die katalytische Methanisierung von CO₂ mit Wasserstoff vorgenommen.

Bei der katalytischen Methanisierung wird das in dem Gasgemisch aus den Massenströmen G1 und G2 enthaltene CO₂ und CO mit dem ebenfalls in dem Gasgemisch enthaltenen Wasserstoff in Methan umgesetzt:

CO₂ + 4 H₂ -> CH₄ + 2 H₂O (1)

CO + 3 H₂-> CH₄ + H₂O (3)

Die beiden Reaktionen werden bei Temperaturen im Bereich von 250°C bis 650°C und Druck im Bereich von 0,1 MPa bis 8 MPa durchgeführt. Bevorzugt wird eine Temperatur im Bereich von 250°C bis 300 °C gewählt. Der Druck wird bevorzugt im Bereich von 2,5 MPa bis 3,5 MPa gewählt und beträgt beispielsweise 3 MPa. Als Katalysator können alle Metalle der achten Nebengruppe des Periodensystems Anwendung finden, bevorzugt wird Nickel eingesetzt. Als Trägermaterial werden bevorzugt aktivierte Aluminium-/Magnesium-Oxide verwendet.

Nach der Umwandlung des im Gasgemisch enthaltenen CO und CO₂ in Methan CH₄ ergibt sich bei Verwendung von reinem Wasserstoff als erster Massenstrom G1 eine Speichereffektivität für den Wasserstoff H₂ von 89 bis 90%.

Bei einer weiteren, nicht erfindungsgemäßen Alternative c) wird eine Direkt-Kohlenstoff-Brennstoffzelle (DCFC) zusammen mit einer CO₂/H₂O Ko-Elektrolyse zur Erzeugung des zweiten Massenstroms G2 eingesetzt.

Hierzu wird eine Direkt-Kohlenstoff-Brennstoffzelle (DCFC) eingesetzt, die Kohlenstoff und Sauerstoff als Edukte in CO₂ umsetzt, wobei Energie in Form von elektrischem Strom freigesetzt wird. Die Brennstoffzelle weist zwei Elektroden auf, die mit einer Membrane, die nur für Sauerstoff-Ionen durchlässig ist, getrennt sind. Der Prozess kann durch folgende Reaktionsgleichungen beschrieben werden:
Kathode:

   O₂ + 4 e- -> 2 O²⁻ (11)
Anode:

   C + 2 O²⁻ -> CO₂ + 4 e- (12)

Derartige Brennstoffzellen werden in dem Artikel K. Hemmes, M. Houwing, N. Woudstra, "Modelling of Direct Carbon Fuel Cell System", J. Fuel Cell Science a. Technology, Vol. 7, Oct. 2010 beschrieben. Die Brennstoffzelle wird bei Temperatur von 700°C bis 900°C, beispielsweise bei 850°C, betrieben. Der elektrische Wirkungsgrad einer solchen Brennstoffzelle liegt üblicherweise bei ca. 80%.

Das aus der Direkt-Kohlenstoff-Brennstoffzelle stammende CO₂ wird anschließend unter Zugabe von Wasserdampf mittels einer Ko-Elektrolyse elektrochemisch zu CO und H₂ umgesetzt. Bevorzugt wird für die Durchführung der Ko-Elektrolyse elektrische Energie und Prozesswärme eingesetzt, die zuvor von der Direkt-Kohlenstoff-Brennstoffzelle erzeugt wurden.

Die bei der Ko-Elektrolyse ablaufenden Reaktionen können wie folgt beschrieben werden:

| | | |
|---|---|---|
| Kathode: | H₂O_{(g)} + CO₂ + 4 e- -> CO + H₂ + 2 O²⁻ | (13) |
| Anode: | 2 O²⁻ +4e- -> O₂ | (14) |

Die CO₂/H₂O-Ko-Elektrolyse wird bevorzugt bei Temperaturen von 700°C bis 950°C durchgeführt, wobei Wasser als Wasserdampf dem Ko-Elektrolyseur zugeführt wird. Zum Erzeugen des Wasserdampfs wird bevorzugt Prozesswärme eingesetzt, die bei der Direkt-Kohlenstoff-Brennstoffzelle oder bei der nachfolgend ausgeführten katalytischen Methanisierungsreaktion anfällt. Der bei der Ko-Elektrolyse anfallende Sauerstoff O₂ wird bevorzugt aufgefangen und als Edukt der Direkt-Kohlenstoff-Brennstoffzelle zugeführt.

Nach dem Herstellen des zweiten Massenstroms G2 wird dieser mit dem ersten Massenstrom G1 vermischt, wobei bevorzugt ein optimales stöchiometrisches Verhältnis zwischen CO und H2, gegebenenfalls mit einem Überschuss von 2 Mol-% bis 5 Mol-% an H2, eingestellt wird. Nach dem Vermischen des erzeugten zweiten Massenstroms G2 mit dem ersten Massenstrom G1 wird die katalytische Methanisierung von CO mit Wasserstoff vorgenommen. Als Produkte der katalytischen Reaktion (3) entstehen Methan und Wasser.

CO + 3 H₂ -> CH₄ + H₂O (3)

Die katalytische Methanisierung und die vorangegangene Ko-Elektrolyse können in zwei separaten Apparaten oder gemeinsam in einem elektrochemischen Reaktor durchgeführt werden.

Der Gesamtprozess lässt sich mit der folgenden elektro-katalytischen Reaktion beschreiben:

C + 2 H₂ -> CH₄ (15)

Der Wirkungsgrad der Reaktion bezogen auf die gespeicherte Wasserstoff-Menge beträgt 100 %, das heißt der über den ersten Massenstrom G1 eingegebene Wasserstoff wird vollständig in Form von Methan gespeichert.

Sowohl bei den erfindungsgemäßen Alternativen a) und b) als auch bei der nicht erfindungsgemäßen Alternative c) ist es bevorzugt, dass die Energiebilanz der Summe der Reaktionen zur Herstellung des Massenstroms G2 und der katalytischen Methanisierungsreaktion positiv eingestellt ist. Hierunter ist zu verstehen, dass in der Summe durch exotherm ablaufende Reaktionen mehr Wärme freigesetzt wird, als zur Aufrechterhaltung von endothermen Reaktionen benötigt wird. Bei der Variante c) wird hierbei bevorzugt über die Direkt-Kohlenstoff-Brennstoffzelle (DCFC) mindestens so viel elektrische Energie erzeugt, wie bei der anschließenden Ko-Elektrolyse benötigt wird. Es ist bekannt, dass das DCFC-Verfahren durch hohe Effizienz gekennzeichnet ist, d.h. bis zu 80% des Kohlenstoffheizwertes wird in elektrische Energie umgewandelt. Dies bedeutet, dass ca. 315 kJ pro Mol C oder CO₂ hergestellt werden. Es ist ebenfalls bekannt, dass die CO₂-Ko-Elektrolyse einen der Wasserelektrolyse ähnlichen Verbrauch der elektrischen Energie aufweist.

Bei der Temperatur von 850°C liegt er standardmäßig bei ca. 190 kJ pro Mol CO₂, oder 225 kJ pro Mol CO₂, wenn man den Wirkungsgrad des Elektrolyseprozesses von 80% berücksichtigt. Da der Energieverlust des DCFC-Prozesses nur ca. 2 bis 3% des Heizwertes des Kohlenstoffs beträgt, deckt die in der exothermen Reaktion generierte Wärme den Ko-Elektrolyse-Bedarf ab. Es stehen ungefähr 17 bis 18 % des Heizwertes in Form von Prozesswärme zur Verfügung, die insbesondere für das Bereitstellen des benötigten Wasserdampfs eingesetzt werden kann.

Bei den Alternativen a) und b) ist es jeweils möglich, zusätzlich zur katalytischen Hydromethanisierung der Variante a) oder zusätzlich zu der Dampf-Wasserstoffvergasung der Variante b) als ein nachfolgender Schritt zur Herstellung des zweiten Massenstroms G2 Kohlendioxid zusammen mit Wasserdampf mittels Ko-Elektrolyse umzusetzen, wobei Kohlenmonoxid und Wasserstoff entsteht.

Kohlenstoffdioxid im zweiten Massenstrom G2 kann elektrochemisch in Synthesegas umgewandelt werden. Hierfür wird CO₂ zusammen mit Wasserdampf unter Einwirkung des elektrischen Stroms in ein Gemisch von Kohlenmonoxid und Wasserstoff überführt. Die Reaktion, bekannt als CO₂/Wasserdampf-Ko-Elektrolyse, wird bevorzugt bei Temperaturen von 600°C bis 1000°C, besonders bevorzugt bei 850°C bis 950°C durchgeführt.

Das bei der katalytischen Hydromethanisierung der Variante a) bzw. bei der Wasserstoffvergasung der Variante b) entstehende CO₂ wird somit in CO umgewandelt, so dass sich die Wasserstoff Speichereffizienz bei der nachfolgenden katalytischen Methanisierungsreaktion verbessert.

Die elektrochemische Umsetzung von CO₂ und Wasserdampf wird wie bei der nicht erfindungsgemäßen Alternative c) bereits beschrieben durch folgende Gleichungen beschrieben.

| | | |
|---|---|---|
| Kathode: | H₂O_{(g)} + CO₂ + 4 e- -> CO + H₂ + 2 O²⁻ | (13) |
| Anode: | 2 O²⁻ + 4 e- -> O₂ | (14) |

Die CO₂/H₂O-Ko-Elektrolyse wird bevorzugt bei Temperaturen von 700°C bis 950°C durchgeführt, wobei Wasser als Wasserdampf dem Elektrolyseur zugeführt wird. Für die Erzeugung des Wasserdampfs wird bevorzugt Prozesswärme eingesetzt, die bei der katalytischen Hydromethanisierung der Variante a) bzw. bei der Dampf-Wasserstoffvergasung der Variante b) und der nachfolgenden katalytischen Methanisierungsreaktion anfällt.

Im Anschluss an die Ko-Elektrolyse wird der zweite Massenstrom G2 mit dem Wasserstoff enthaltenden ersten Massenstrom G1 vermischt und die katalytische Methanisierungsreaktion wie zuvor beschrieben durchgeführt.

Wendet man die Hydromethanisierung gemäß Alternative a) in Kombination mit der Ko-Elektrolyse an, ergibt sich die folgende elektro-katalytische Gesamtreaktion:

6,1 C + 6 H₂O + 6,2 H₂ -> 6,1 CH₄ + 3O₂ (16)

Der Wirkungsgrad der Reaktion bezogen auf die im ersten Massenstrom enthaltene Wasserstoffmenge beträgt 196 %, wobei für die Ko-Elektrolyse zusätzliche Energie in Form des bei der Ko-Elektrolyse eingesetzten elektrischen Stroms notwendig ist. Setzt man die Ko-Elektrolyse einer zur Herstellung von Wasserstoff durchgeführten Wasser-Elektrolyse gleich und schlägt diesen für die Berechnung des Wirkungsgrads dem ersten Massenstrom G1 zu, reduziert sich der Wirkungsgrad auf 131 %.

Sowohl bei den erfindungsgemäßen Alternativen a) und b) als auch bei der nicht erfindungsgemäßen Alternative c) des Verfahrens entsteht bei der katalytischen Methanisierungsreaktion Wasserdampf. Bevorzugt wird der Wasserdampf bzw. das Wasser aufgefangen und zurückgeführt, so dass dieses bei der Herstellung des zweiten Massenstroms G2 als Edukt verwendet wird.

### Vorteile der Erfindung

Mittels des erfindungsgemäß vorgeschlagenen Verfahrens kann bei der Speicherung von Wasserstoff durch Erzeugung von Methan die Speichereffizienz gegenüber den bekannten Verfahren gesteigert werden. Dies wird dadurch erreicht, dass der bei der katalytischen Methanisierungsreaktion auftretende Verlust von Wasserstoff durch das Entstehen von Wasserdampf durch zusätzliche durchgeführte Reaktionen, bei denen Methan entsteht, ausgeglichen wird. Alternativ oder zusätzlich wird die Speichereffizienz der katalytischen Methanisierungsreaktion dadurch gesteigert, dass der für die Methanisierungsreaktion erforderliche Kohlenstoff bevorzugt in Form von Kohlenmonoxid bereitgestellt wird. Bei der Methanisierung von Wasserstoff mittels Kohlenmonoxid geht weniger Wasserstoff in Form von Wasserdampf verloren als bei der Verwendung von Kohlendioxid.

In bevorzugten Ausführungsformen werden die Reaktionsbedingungen der bei dem Verfahren ablaufenden Reaktionen so eingestellt, dass die Energiebilanz in der Summe positiv ist.

Somit muss vorteilhafterweise für die Erzeugung des Methans keine Energie in Form von Prozesswärme oder elektrischer Energie von außen zugeführt werden. Eine Ausnahme sind hier Ausführungsformen der Varianten a) und b), bei denen zusätzlich eine Ko-Elektrolyse durchgeführt wird. Hierbei muss dem Verfahren zusätzliche Energie in Form von elektrischem Strom zugeführt werden. Der bei dem Ko-Elektrolyse-Verfahren erforderliche Außeneintrag elektrischer Energie wird jedoch durch die besonders hohe Wasserstoffausbeute kompensiert. Das erfindungsgemäße Verfahren ermöglicht es, den zur Verfügung stehenden, zu speichernden Wasserstoff in einem energieneutralen Prozess nahezu vollständig in Methan zu überführen und zu speichern.

Der zu speichernde Wasserstoff kann dabei nicht erfindungsgemäß durch ein Elektrolyse-Verfahren unter

Anwendung von erneuerbaren Energien bereitgestellt werden. Das erfindungsgemäße Verfahren sieht dagegen vor, an Wasserstoff reiche Gasgemische als Wasserstoffquelle einzusetzen, vorteilhafterweise solche, die häufig als Abfall in industriellen Prozessen entstehen. Beispielsweise fällt bei der Chlor-Alkali-Elektrolyse oder bei der Koks-Herstellung ein wasserstoffreiches Gas (G1) an, welches im Zusammenhang mit dem erfindungsgemäßen Verfahren verwendet werden kann. Bei der Verwendung von solchen Abfallprodukten als ersten Massenstrom G1 wird vorteilhafterweise ein Abfallprodukt aufgewertet, so dass dieses als wertvoller Energieträger zur Verfügung steht.

Das vorgeschlagene Verfahren ermöglicht eine effiziente Verwertung von Abfallprodukten wie Kokereigas. Kokereigase werden aktuell in erster Linie thermisch zu verwertet. In dem vorgeschlagenen Verfahren wird hingegen eine stoffliche Verwertung realisiert und somit werden wertvolle chemische Rohstoffe wie z.B. Wasserstoff und Kohlenmonoxid wiedergewonnen, die üblicherweise aus fossilen Rohstoffen hergestellt werden würden. Kurze Beschreibung der Figuren

Ausführungsbeispiele sind in den Zeichnungen dargestellt und in der nachfolgenden Beschreibung näher erläutert.
Figur 1 zeigt eine schematische Darstellung einer nicht erfindungsgemäßen Ausführungsform des Verfahrens, bei dem als erster Massenstrom G1 Wasserstoffgas eingesetzt wird,
Figur 2 zeigt eine schematische Darstellung der ersten erfindungsgemäßen Ausführungsform des Verfahrens, wobei Koksofengas als erster Massenstrom G1 eingesetzt wird,
Figur 3 zeigt schematisch eine zweite Ausführungsform des Verfahrens,
Figur 4 zeigt eine schematische Darstellung einer dritten Ausführungsform des Verfahrens und
Figur 5 zeigt eine weitere, nicht erfindungsgemäße Ausführungsform des Verfahrens in schematischer Darstellung.

In der nachfolgenden Beschreibung der Ausführungsbeispiele der Erfindung werden gleiche Komponenten und Elemente mit gleichen Bezugszeichen bezeichnet, wobei auf eine wiederholte Beschreibung dieser Komponenten oder Elemente in Einzelfällen verzichtet wird. Die Figuren stellen den Gegenstand der Erfindung nur schematisch dar.

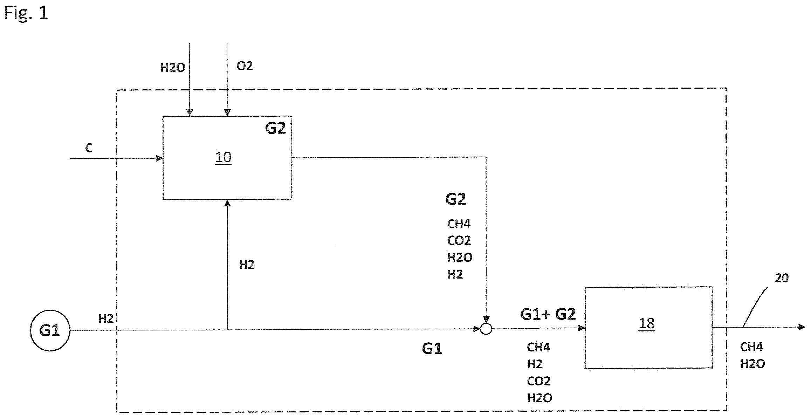

Figur 1 zeigt schematisch eine nicht erfindungsgemäße Ausführungsform des Verfahrens bei dem als ein erster Massenstrom G1 Wasserstoffgas verwendet wird. Zur Herstellung eines zweiten Massenstroms G2 wird eine katalytische Hydromethanisierung 10 eingesetzt. Als Edukte für die katalytische Hydromethanisierung 10 werden Kohlenstoff C, Wasser H₂O und Sauerstoff O₂ zugeführt. Zum Einstellen des Reaktionsgleichgewichts wird zusätzlich Wasserstoff H₂ aus dem ersten Massenstrom G1 zugeführt. Hierzu wird der erste Massenstrom G1 aufgeteilt, wobei ein Teilstrom der katalytischen Hydromethanisierung 10 zugeleitet wird.

Die katalytische Hydromethanisierung 10 kann beispielsweise in einem Reaktor ausgeführt werden, in dem eine Mischung aus Katalysator und Kohlenstoff in fester Form, beispielsweise in Form von Ruß, enthalten ist. In den Reaktor wird Wasser in Form von Wasserdampf sowie eine geringe Menge Sauerstoff eingebracht. In Gegenwart des Katalysators reagiert der Kohlenstoff mit dem Wasserdampf zu Methan und Kohlendioxid. Die Reaktion wird bei einem Druck im Bereich von 3 MPa bis 7 MPa und einer Temperatur im Bereich von 400 °C bis 800 °C ausgeführt. Die Reaktion weist eine neutrale oder positive Energiebilanz auf, so dass zum Aufrechterhalten der Reaktion keine Energie zugeführt werden muss. Die gasförmigen Produkte werden als zweiter Massenstrom G2 entnommen, wobei zuvor gegebenenfalls enthaltene Partikel, beispielsweise Katalysatorpartikel, abgetrennt werden. Zum Abtrennen der festen Partikel kann beispielsweise ein Zyklon eingesetzt werden.

Der im Reaktor enthaltene Katalysator wird von Zeit zu Zeit regeneriert und der regenerierte Katalysator wird nach Zugabe von Ruß erneut in den Reaktor eingegeben. Als Katalysator wird bevorzugt ein alkalischer Katalysator bestehend aus einer Mischung mindestens zweier Substanzen aus der Gruppe Na₂CO₃, K₂CO₃, Rb₂CO₃, Li₂CO₃, Cs₂CO₃, KNO₃, K₂SO₄, NaOH, KOH oder Mineralien, die diese Substanzen enthalten, verwendet. Zusätzlich als CO2-Binder werden Kalziumverbindungen wie CaO oder Ca(OH)₂ beigemischt. Die Reaktion wird vorzugsweise in einem Wirbelbettreaktor durchgeführt.

Der mittels katalytischer Hydromethanisierung erzeugte zweite Massenstrom G2 enthält Methan CH₄, Kohlendioxid CO₂, Wasserdampf H₂O und in der dargestellten Ausführungsform zusätzlich Wasserstoff H₂. Der zweite Massenstrom G2 wird mit dem verbleibenden Teil des ersten Massenstrom G1 vermischt und einer katalytischen Methanisierung 18 zugeführt. Die Mischung aus dem ersten Massenstrom G1 und dem zweiten Massenstrom G2 enthält Methan CH₄, Kohlendioxid CO₂, Wasserstoff H₂ und Wasserdampf H₂O, wobei bei dem Mischvorgang die Mengen des ersten Massenstroms G1 und des zweiten Massenstroms G2 bevorzugt so gewählt sind, dass ein stöchiometrisches Verhältnis von vier Molekülen H₂ auf ein Molekül CO₂ eingestellt wird. Gegebenenfalls wird das stöchiometrische Verhältnis so eingestellt, dass ein Überschuss von 2% bis 5% an Wasserstoff vorhanden ist. In dem gemischten Massenstrom enthaltenes Kohlendioxid CO₂ wird bei der katalytischen Methanisierung 18 zu Methan hydriert.

Die katalytische Methanisierung 18 erfolgt in einem Reaktor in Gegenwart eines Katalysators bevorzugt bei Temperaturen im Bereich von 250°C bis 650°C und Druck im Bereich von 0,1 MPa bis 8 MPa. Als Katalysator können alle Metalle der achten Nebengruppe des Periodensystems Anwendung finden. Als Trägermaterial für den Katalysator werden bevorzugt aktivierte Aluminium-/Magnesium-Oxide verwendet. Bevorzugt wird die Reaktion isotherm z.B. in einem Plattenreaktor, der durch sehr gute Wärmeübertragungsfähigkeit verfügt, durchgeführt.

Als Endprodukt 20 entsteht bei der katalytischen Methanisierung 18 ein Gasgemisch, welches Methan CH₄ und Wasserdampf H₂O enthält. Der Wasserdampf H₂O wird bevorzugt durch Kondensieren des Wassers abgetrennt, so dass Methan CH₄ mit hoher Reinheit bereitgestellt wird.

Das Methan CH₄ kann anschließend beispielsweise in ein öffentliches Gasnetz eingespeist werden oder anderweitig genutzt werden.

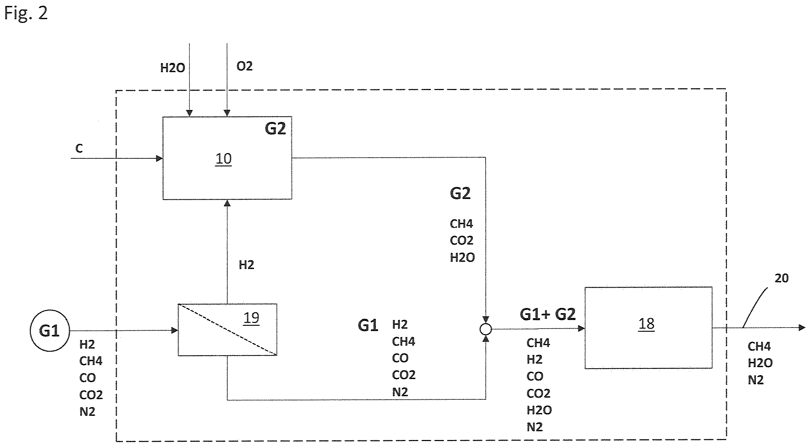

Figur 2 stellt wie Figur 1 schematisch eine erste, erfindungsgemäße Ausführungsform des Verfahrens dar, jedoch wird abweichend von der Darstellung der Figur 1 Kokereigas (KG) als erster Massenstrom G1 verwendet. Der erste Massenstrom G1 ist reich an Wasserstoff H₂, enthält jedoch zusätzlich Methan CH₄, Kohlendioxid CO₂, Kohlenmonoxid CO und Stickstoff N₂.

Bei der Herstellung des zweiten Massenstroms G2 wird wie mit Bezug zur Figur 1 bereits beschrieben eine katalytische Hydromethanisierung 10 eingesetzt. Zum Einstellen des Reaktionsgleichgewichts wird zusätzlich Wasserstoff H₂ aus dem ersten Massenstrom G1 zugeführt. Für diese Zwecke wird Wasserstoff aus den Strom G1 teilweise abgetrennt. Hierfür wird beispielsweise Druckwechseladsorption 19 eingesetzt. Die anderen Komponenten des KG-Gasgemisches sowie der restliche Wasserstoff bleiben im ersten Massenstrom G1.

Nach dem Erzeugen des Massenstroms G2 wird dieser mit dem verbleibenden Teil des ersten Massenstroms G1 vermischt und es wird wie mit Bezug zur Figur 1 bereits beschrieben die katalytische Methanisierung 18 durchgeführt. In dem gemischten Massenstrom enthaltenes Kohlendioxid CO₂ und Kohlenmonoxid CO wird dabei jeweils zu Methan hydriert.

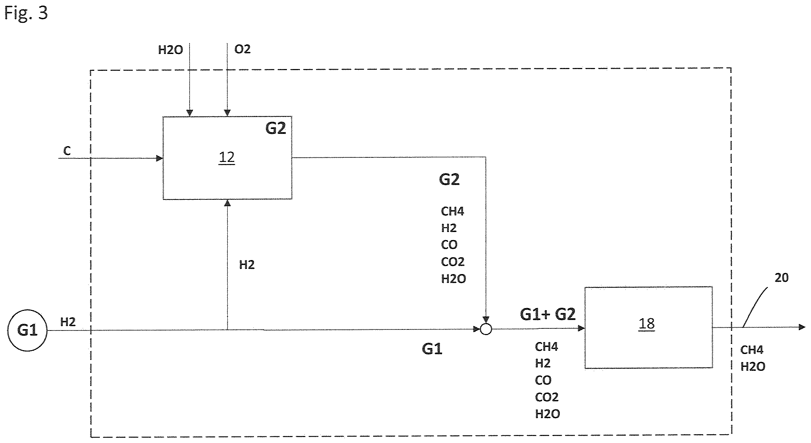

Figur 3 zeigt schematisch eine zweite Ausführungsform des Verfahrens bei dem als ein erster, nicht erfindungsgemäßer Massenstrom G1 Wasserstoffgas verwendet wird. Erfindungsgemäß wird das Verfahren der zweiten Ausführungsform jedoch mit einem an Wasserstoff reichen Gasgemisch wie beispielsweise Kokereigas (KG) durchgeführt.

Zur Herstellung eines zweiten Massenstroms G2 wird eine Dampf-Wasserstoffvergasung 12 durchgeführt. Als Edukte für die Dampf-Wasserstoffvergasung 12 werden Kohlenstoff C, Wasser H₂O und Sauerstoff O₂ zugeführt. Zum Einstellen des Reaktionsgleichgewichts wird zusätzlich Wasserstoff H₂ aus dem ersten Massenstrom G1 zugeführt. Hierzu wird der erste Massenstrom G1 aufgeteilt, wobei ein Teilstrom der katalytischen Hydromethanisierung 10 zugeleitet wird.

Die Dampf-Wasserstoffvergasung 12 wird in einem Reaktor bei einem Druck Im Bereich von 3 MPa bis 7 MPa, und einer Temperatur im Bereich von 800 °C bis 1100°C ausgeführt. Hierbei wird ein Teil des Kohlenstoffs verbrannt und gleichzeitig Wasser in Wasserstoff gespalten. Als Produkte entstehen hierbei Methan CH₄, Wasserstoff H₂, Kohlendioxid CO₂ und Kohlenmonoxid CO. Des Weiteren sind im entstehenden zweiten Massenstrom G2 noch Teile des Wasserdampfs H₂O enthalten. Die Ausführung des Reaktors kann beispielsweise als Festbett- oder als Wirbelschichtbett-Apparat erfolgen, wobei Sauerstoff und Wasserdampf am Boden des Reaktors eingeleitet werden. Darüber in ca. 15% der Reaktorhöhe wird Wasserstoff über einen Verteiler eingeführt. Die Beschickung mit Kohlenstoff erfolgt am Dom des Reaktors.

Der mittels der Dampf-Wasserstoffvergasung 12 erzeugte zweite Massenstrom G2 wird mit dem verbleibenden Teil des ersten Massenstrom G1 vermischt und der katalytischen Methanisierung 18 zugeführt. Die Mischung aus dem ersten Massenstrom G1 und dem zweiten Massenstrom G2 enthält Methan CH₄, Kohlenmonoxid CO, Kohlendioxid CO₂, Wasserstoff H₂ und Wasserdampf H₂O, wobei bei dem Mischvorgang die Mengen des ersten Massenstroms G1 und des zweiten Massenstroms G2 bevorzugt so gewählt sind, dass ein stöchiometrisches Verhältnis von vier Molekülen H₂ auf ein Molekül CO₂ und von drei Molekülen H₂ auf ein Molekül CO eingestellt wird. Gegebenenfalls wird das stöchiometrische Verhältnis so eingestellt, dass ein Überschuss von 2% bis 5% an Wasserstoff vorhanden ist. In dem gemischten Massenstrom enthaltenes Kohlendioxid CO₂ und Kohlenmonoxid CO wird bei der anschließend durchgeführten katalytischen Methanisierung 18 zu Methan hydriert.

Das Endprodukt 20 ist wiederum ein an Methan reiches Gas, welches noch Wasserdampf H₂O enthält. Der Wasserdampf wird bevorzugt mittels Kondensation des Wassers abgetrennt, so dass Methangas mit hoher Reinheit zur Verfügung steht.

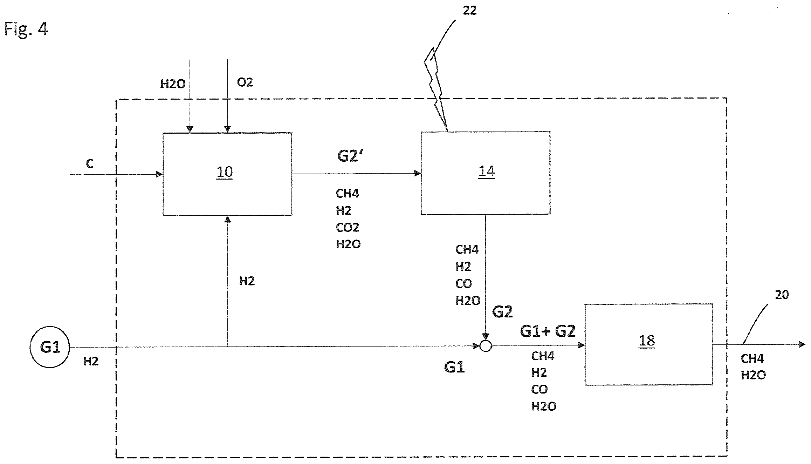

Figur 4 stellt eine dritte Ausführungsform des Verfahrens schematisch dar. Als erster Massenstrom G1 wird in dem dargestellten Beispiel nicht erfindungsgemäß Wasserstoffgas eingesetzt, gemäß der Erfindung wird jedoch ein an Wasserstoff reiches Gasgemisch wie beispielsweise Kokereigas eingesetzt.

Bei der zweiten Ausführungsform des Verfahrens erfolgt, wie mit Bezug zu der Figur 1 beschrieben, eine katalytische Hydromethanisierung 10. Im Gegensatz zur ersten Ausführungsform der Figur 1 wird bei der zweiten Ausführungsform des Verfahrens mittels der katalytischen Hydromethanisierung 10 ein Zwischenprodukt G2' erzeugt, indem die Edukte Kohlenstoff C, Wasserdampf H₂O und Sauerstoff O₂ zu Methan CH₄ und Kohlendioxid CO₂ umgesetzt werden. Das Zwischenprodukt G2' enthält zudem noch Teile des als Edukts eingesetzten Wasserdampfs sowie einen Teil des zur Einstellung des Reaktionsgleichgewichts aus dem ersten Massenstrom G1 zugeführten Wasserstoffs.

Alternativ ist es möglich, die mit Bezug zur Figur 3 beschriebene Dampf-Wasserstoffvergasung 12 zur Erzeugung des Zwischenprodukts G2' einzusetzen.

Das Zwischenprodukt G2' wird in einer CO₂/H₂O Ko-Elektrolyse zur Erzeugung des zweiten Massenstroms G2 eingesetzt. Die CO₂/H₂O-Ko-Elektrolyse wird bevorzugt bei Temperaturen von 700°C bis 950°C durchgeführt, wobei Wasserdampf H₂O und Kohlendioxid CO₂ zu Wasserstoff H₂, Kohlenmonoxid CO und Sauerstoff O₂ umgesetzt werden. Der bei der Ko-Elektrolyse anfallende Sauerstoff wird nicht dem zweiten Massenstrom G2 zugegeben. Zur Durchführung der Ko-Elektrolyse wird elektrischer Strom 22 eingesetzt, der bevorzugt aus erneuerbaren Energiequellen gewonnen wird.

Der aus dem Zwischenprodukt G2' erzeugte zweite Massenstrom G2 enthält Methan CH4, Wasserstoff H2 und Kohlenmonoxid CO. Zusätzlich können bei der Ko-Elektrolyse nicht umgesetzte Reste an Wasserdampf H₂O noch im zweiten Massenstrom G2 enthalten sein. Der zweite Massenstrom G2 wird mit dem ersten Massenstrom G1 vermischt, wobei bei dem Mischvorgang die Mengen des ersten Massenstroms G1 und des zweiten Massenstroms G2 bevorzugt so gewählt sind, dass ein stöchiometrisches Verhältnis von drei Molekülen H₂ auf ein Molekül CO eingestellt wird. Gegebenenfalls wird das stöchiometrische Verhältnis so eingestellt, dass ein Überschuss von 2 Mol-% bis 5 Mol-% an Wasserstoff vorhanden ist. In dem gemischten Massenstrom enthaltenes Kohlenmonoxid CO wird bei der anschließend durchgeführten katalytischen Methanisierung 18 zu Methan CH₄ hydriert.

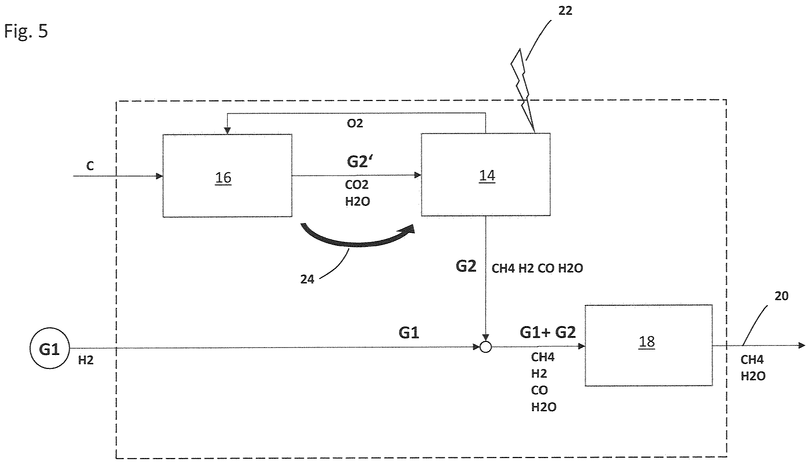

In Figur 5 ist eine weitere, nicht erfindungsgemäße Ausführungsform des Verfahrens schematisch dargestellt. Als erster Massenstrom G1 wird in dem dargestellten Beispiel Wasserstoffgas eingesetzt, es könnte jedoch alternativ auch ein an Wasserstoff reiches Gasgemisch wie beispielsweise Kokereigas eingesetzt werden.

Zur Herstellung des zweiten Massenstroms G2 wird Kohlenstoff C zusammen mit Sauerstoff 02 als Edukte in eine Direkt-Kohlenstoff-Brennstoffzelle 16 eingegeben und in Kohlendioxid CO₂ umgesetzt, wobei Energie 24 in Form von Prozesswärme und elektrischem Strom freigesetzt wird. Das Zwischenprodukt G2' wird einer CO₂/H₂O Ko-Elektrolyse zur Erzeugung des zweiten Massenstroms G2 zugeführt. Die für die Elektrolyse benötigte elektrische Energie wird zumindest teilweise über die in der Direkt-Kohlenstoff-Brennstoffzelle 16 freigesetzte Energie 24 bereitgestellt. Sofern die freigesetzte Energie 24 nicht zum Betreiben der Elektrolyse ausreichend ist, wird zusätzliche elektrische Energie 22 zugegeben, die bevorzugt aus erneuerbaren Energien stammt.

Bei der CO₂/H₂O Ko-Elektrolyse werden Kohlendioxid CO₂ und Wasserdampf H₂O in Wasserstoff H2, Kohlenmonoxid CO und Sauerstoff O₂ umgesetzt, wobei der Sauerstoff O₂ aufgefangen wird und als Edukt der Direkt-Kohlenstoff-Brennstoffzelle 16 zugeführt wird. Wasserstoff H2 und Kohlenmonoxid CO werden ebenfalls aufgefangen und als zweiter Massenstrom G2 mit dem ersten Massenstrom G1 vermischt, wobei bei dem Mischvorgang die Mengen des ersten Massenstroms G1 und des zweiten Massenstroms G2 bevorzugt so gewählt sind, dass ein stöchiometrisches Verhältnis von drei Molekülen H₂ auf ein Molekül CO eingestellt wird, gegebenenfalls mit einem Überschuss von 2% bis 5% an H₂.

Im Anschluss an das Mischen wird die katalytische Methanisierung 18 wie mit Bezug zu den Figuren 1 bis 4 bereits beschrieben ausgeführt, wobei Kohlenmonoxid CO zu Methan CH₄ hydriert wird.

Die Erfindung ist nicht auf die hier beschriebenen Ausführungsbeispiele und die darin hervorgehobenen Aspekte beschränkt. Vielmehr ist innerhalb des durch die Ansprüche angegebenen Bereichs eine Vielzahl von Abwandlungen möglich, die im Rahmen fachmännischen Handelns liegen.

### Bezugszeichenliste

- G1: erster Massenstrom
- G2: zweiter Massenstrom

- G2': Zwischenprodukt

- 10: katalytische Hydromethanisierung
- 12: Dampf-Wasserstoffvergasung
- 14: Ko-Elektrolyse
- 16: Kohlenstoff-Direktbrennstoffzelle
- 18: katalytische Methanisierung
- 19: Druckwechseladsorption
- 20: Endprodukt
- 22: elektrischer Strom
- 24: freigesetzte Energie

## Patentansprüche

1. Verfahren zur Herstellung von Methan, wobei ein erster Massenstrom G1, der ein Wasserstoff enthaltenes Gasgemisch ist, mit einem zweiten Massenstrom G2, der Kohlendioxid und/oder Kohlenmonoxid enthält, vermischt wird und Wasserstoff sowie Kohlendioxid und/oder Kohlenmonoxid im erhaltenen Gemisch in einer katalytischen Reaktion in Methan umgesetzt wird, wobei der zweite Massenstrom G2 erhalten wird durch
a) Durchführen einer katalytischen Hydromethanisierung, bei der Kohlenstoff, Wasserdampf und Sauerstoff als Edukte zu Methan, Kohlenmonoxid und Kohlendioxid bei einer Temperatur im Bereich von 400 °C bis 800 °C in Gegenwart mindestens eines Katalysators umgesetzt werden, oder
b) Durchführen einer Dampf-Wasserstoffvergasung, bei der Kohlenstoff, Wasserstoff, Wasserdampf und Sauerstoff als Edukte zu Methan, Kohlenmonoxid und Kohlendioxid bei einer Temperatur im Bereich von 800 °C bis 1100 °C umgesetzt werden
wobei bei der katalytischen Hydromethanisierung der Alternative a) oder bei der Dampf-Wasserstoffvergasung gemäß Alternative b) Wasserstoff als zusätzliches Edukt zugegeben wird, um das Reaktionsgleichgewicht zugunsten des Methans einzustellen, wobei der Wasserstoff aus dem ersten Massenstrom G1 abgetrennt wird.

2. Verfahren nach Anspruch 1, **dadurch gekennzeichnet, dass** bei der katalytischen Hydromethanisierung der Alternative a) der Druck im Bereich von 5,5 MPa bis 6,5 MPa liegt.

3. Verfahren nach Anspruch 1 oder 2, **dadurch gekennzeichnet, dass** bei der katalytischen Hydromethanisierung der Alternative a) die Temperatur im Bereich von 500°C bis 600°C liegt.

4. Verfahren nach Anspruch 1, **dadurch gekennzeichnet, dass** bei der Dampf-Wasserstoffvergasung der Alternative b) der Druck im Bereich von 4,5 MPa bis 5,5 MPa liegt.

5. Verfahren nach Anspruch 1 oder 4, **dadurch gekennzeichnet, dass** bei der Dampf-Wasserstoffvergasung der Alternative b) die Temperatur im Bereich von 900°C bis 1000°C liegt.

6. Verfahren nach einem der Ansprüche 1 bis 5, **dadurch gekennzeichnet, dass** der Massenstrom G1 Abfallprodukt einer anderen Reaktion ist.

7. Verfahren nach einem der Ansprüche 1 bis 6, **dadurch gekennzeichnet, dass** bei der katalytischen Methanisierungsreaktion entstehendes Wasser zurückgeführt wird und als Edukt bei der Herstellung des Massenstroms G2 eingesetzt wird.

## Claims

1. Process for producing methane, wherein a first mass flow G1, which is a hydrogen-containing gas mixture, is mixed with a second mass flow G2, which contains carbon dioxide and/or carbon monoxide, and hydrogen and carbon dioxide and/or carbon monoxide in the obtained mixture are converted into methane in a catalytic reaction, wherein the second mass flow G2 is obtained by
a) performing a catalytic hydromethanation in which the inputs carbon, steam and oxygen are converted in the presence of at least one catalyst at a temperature in the range from 400°C 800°C into methane, carbon monoxide and carbon dioxide or
b) performing a steam-hydrogen gasification in which the inputs carbon, hydrogen, steam and oxygen are converted at a temperature in the range from 800°C to 1100°C into methane, carbon monoxide and carbon dioxide
wherein in the catalytic hydromethanation of alternative a) or in the steam-hydrogen gasification according to alternative b) hydrogen is added as an additional input to adjust the reaction equilibrium in favour of methane, wherein the hydrogen is separated from the first mass flow G1.

2. Process according to Claim 1, **characterized in that** in the catalytic hydromethanation of alternative a) the pressure is in the range from 5.5 MPa to 6.5 MPa.

3. Process according to Claim 1 or 2, **characterized in that** in the catalytic hydromethanation of alternative a) the temperature is in the range from 500°C to 600°C.

4. Process according to Claim 1, **characterized in that** in the steam-hydrogen gasification of alternative b) the pressure is in the range from 4.5 MPa to 5.5 MPa.

5. Process according to Claim 1 or 4, **characterized in that** in the steam-hydrogen gasification of alternative b) the temperature is in the range from 900°C to 1000°C.

6. Process according to any of Claims 1 to 5, **characterized in that** the mass flow G1 is the waste product of another reaction.

7. Process according to any of Claims 1 to 6, **characterized in that** water formed in the catalytic methanation reaction is recycled and used as an input in the production of the mass flow G2.

## Revendications

1. Procédé de production de méthane, dans lequel un premier courant massique G1, qui est un mélange gazeux contenant de l'hydrogène, est mélangé à un deuxième courant massique G2, qui contient du dioxyde de carbone et/ou du monoxyde de carbone, et l'hydrogène ainsi que le dioxyde de carbone et/ou le monoxyde de carbone dans le mélange obtenu sont convertis en méthane dans une réaction catalytique, le deuxième courant massique G2 étant obtenu par
a) réalisation d'une hydrométhanisation catalytique, lors de laquelle du carbone, de la vapeur d'eau et de l'oxygène sont convertis en tant que produits de départ en méthane, monoxyde de carbone et dioxyde de carbone à une température dans la plage allant de 400 °C à 800 °C en présence d'au moins un catalyseur, ou
b) réalisation d'une gazéification vapeur-hydrogène, lors de laquelle du carbone, de l'hydrogène, de la vapeur d'eau et de l'oxygène sont convertis en tant que produits de départ en méthane, monoxyde de carbone et dioxyde de carbone à une température dans la plage allant de 800 °C à 1 100 °C,
dans lequel, lors de l'hydrométhanisation catalytique de la variante a) ou lors de la gazéification vapeur-hydrogène selon la variante b), de l'hydrogène est ajouté en tant que produit de départ supplémentaire, afin d'ajuster l'équilibre réactionnel en faveur du méthane, l'hydrogène étant séparé du premier courant massique G1.

2. Procédé selon la revendication 1, **caractérisé en ce que**, lors de l'hydrométhanisation catalytique de la variante a), la pression se situe dans la plage allant de 5,5 MPa à 6,5 MPa.

3. Procédé selon la revendication 1 ou 2, **caractérisé en ce que**, lors de l'hydrométhanisation catalytique de la variante a), la température se situe dans la plage allant de 500 °C à 600 °C.

4. Procédé selon la revendication 1, **caractérisé en ce que**, lors de la gazéification vapeur-hydrogène de la variante b), la pression se situe dans la plage allant de 4,5 MPa à 5,5 MPa.

5. Procédé selon la revendication 1 ou 4, **caractérisé en ce que**, lors de la gazéification vapeur-hydrogène de la variante b), la température se situe dans la plage allant de 900 °C à 1 000 °C.

6. Procédé selon l'une quelconque des revendications 1 à 5, **caractérisé en ce que** le courant massique G1 est un déchet d'une autre réaction.

7. Procédé selon l'une quelconque des revendications 1 à 6, **caractérisé en ce que** l'eau produite lors de la réaction de méthanisation catalytique est recyclée et utilisée en tant que produit de départ dans la production du courant massique G2.
